# EUROPEAN PATENT APPLICATION

(11) **EP 3 957 726 A1**
(43) Date of publication of application: **23.02.2022**
(21) Application number: 20192187.1
(22) Date of filing: 21.08.2020
(51) Int. Cl.: C12N 9/10, C12N 15/63, C12N 15/82

(54) **BIOSYNTHESIS AND METHOD OF PRODUCTION OF PSILOCIN AND PSILOCYBIN IN MICROORGANISMS**

(71) Applicant: Synbionik GmbH, 60314 Frankfurt am Main (DE)
(72) Inventor: TABOADA, Andrea, 60439 Frankfurt am Main (DE); SCHMITT, Patrick, 55122 Mainz (DE); SCHOEFFER, Henrik, 63069 Offenbach (DE)
(74) Representative: Kilger, Ute

(57) **Abstract**

The following invention describes generally to methods of production of psilocin and psilocybin, as well as expression of enzymes and recombinant microorganisms for the biosynthesis of pharmaceutical approved psilocin and psilocybin. Using DMT as starting material, heterologous enzymes are used to direct psilocin and psilocybin biosynthesis in modified microorganisms, e.g., bacteria, yeasts.

### SEQUENCES

<110> Synbionik GmbH
<120> Biosynthesis and Method of Production of Psilocin and Psilocybin in Microorganisms
<130> SynPsy
<160> 3
<170> BiSSAP 1.3.6
<210> 1
   <211> 1634
   <212> DNA
   <213> Gymnopilus dilepis
<220>
   <223> Putative Dimethyltryptamine 4-hydroxylase
<400> 1 
ccatgggccg tcgggcaaga agggctcgac tacccccggg tccaccgggt atcccgttgc 60
catttgtggg taatctgttt gatatgccgt ctaacagccc gtggctgacc tatctgcaat 120
ggggtgagac gtatcagacc gacattatct acctgaatgc gggtggcacc gagatggtga 180
ttcttaacac tttggaggcg attaccgatc tgttggagaa gagaggctcc atctatagcg 240
gtcgttttga aagcacgatg gtgaacgaat taatgggttg ggattttgac ttgggcttta 300
tcacgtacgg cgaacgctgg cgtgaggaac gtcgcatgtt cagcaaagag ttcaacgaga 360
agaacattaa gcagtttcgt cacgcccaga tccgtgctgc gaacttgctg gtcggccagc 420
tgaccaaaac cccggagcgt tggcatcaac tgatccgcca ccaaatagcg gcgatgtccc 480
tggatatcgg ttacggcatt gatctcttgg agggtgatcc gtggctggag gcgacccagt 540
tggcgaatga gggcctggcg atagcgagcg ttccgggtag cttctgggtt gattccctgc 600
cgattttaaa atacatgccc tcctggtttc cgggcgccga gttcaagcgc aaggcgaaag 660
tttggcgtga gagcaccgac cacatgatta acatgccgta cgaaaagatg aaaaagctga 720
 
tggttcaaga tttggttcgt ccgagttacg caagcgcgcg tctgcaagag atggacccga 780
atggcgatct gcagcatcaa gaacatgtta tccgtaacac cgcaatggaa gttaatgttg 840
gtggtgctga cactacagtg agcgcagtgg cggcgttcat cttagcgatg gtgaaatacc 900
cggacgttca gcgtaaggtg caggcagaac tggacgcggt tggctgtcgt gatgagctgc 960
cggagttcga cgaggacaac gatgcactgc cgtacctgac ggcgtgcgtt aaagaaattt 1020
ttcgttggaa tcaggtcgct ccgctggcca tcccgcaccg tctggacaaa gatgatcact 1080
atcgtggtta catcattccg aaaaacgcgc tggtgttcgc taatacttgg gcagtcctga 1140
atgacccatc ggtgtatccg gacccgtctg agttccgtcc ggagcgttat ctgggcccag 1200
acggcaagcc ggacccgcgt attcgcgacc cgcgtaaggc ggcgtttggt tatggcagac 1260
gcgcttgccc gggcatccac ctagctcaaa gcacggtgtg gattgttggt gctaccctgc 1320
tgtccgtgtt cgacatcgaa cgccctatgg atgcgaacgg taaaccgatt gacatcccag 1380
ctgcattcac caccggtttc tttcgctatt ctattcatga ttgtctcgtg gtggaaacga 1440
tgcacccggc gaacaccgtt tgcgtcgaca tcccgaaccc gtcggatgcc gatagcttcc 1500
tggtgccgaa gcgcctgtcg aacccgcatc cgattatcga cttgccgagc cgaaatccgg 1560
cgtgccagga agacggcgta gtggccttgt ctaacgcctg gcgcagcacc ttgccggttc 1620
aagacgtgaa gctt 1634
<210> 2
   <211> 1481
   <212> DNA
   <213> Psilocybe cubensis
<220>
   <223> Tryptamine 4-monooxygenase
<400> 2 
ccatgggccg tcgggtacga aggtcaagac taccccctgg accgcctggc atcccgattc 60
catttatcgg taatatgttt gatatgcccg aggaaagccc gtggctgacc tttctgcaat 120
ggggtcgtga ttacaacacg gacatcctgt atgtggacgc gggcggtaca gaaatggtca 180
tcttgaacac cctggaaacc attaccgatt tgttggagaa acgcggtagc atttattccg 240
 
gcagattgga gagcacgatg gtgaacgagt tgatgggttg ggagttcgac ctgggcttca 300
ttacgtacgg cgaccgctgg cgtgaagagc gccgcatgtt cgccaaagag ttcagcgaaa 360
agggtatcaa acagtttcgt cacgcacaag taaaggcggc acatcaactg gtgcagcagt 420
tgaccaaaac gccggaccgc tgggcgcaac atattcgtca ccaaatcgct gccatgtctc 480
tcgatatcgg ctacggcatc gacttggcgg aagacgaccc gtggcttgag gcaacccatc 540
tggcgaatga aggtctggcg attgcgagcg ttccaggcaa gttctgggtc gactcgtttc 600
cgagcttaaa atacttaccg gcatggtttc cgggtgcggt gttcaaacgt aaggcgaaag 660
tttggcgtga agcagctgac cacatggtgg acatgccgta cgaaaccatg cgtaagctgg 720
ctccgcaggg tctgacccgt ccgagctatg caagcgcgcg tctccaggcg atggatctca 780
acggcgatct ggagcaccag gaacacgtga ttaagaacac tgcggcggag gttaatgttg 840
gtggcggtga taccaccgtt tccgccatgt cagcgttcat cctggctatg gtgaagtacc 900
cggaagtgca acgtaaggtt caagcggagc tggatgctct gacaaataac ggtcagatcc 960
cggactacga cgaagaggac gatagcctgc cgtatttgac tgcgtgtatt aaggagttgt 1020
ttcgctggaa tcagatcgca ccgctggcca tcccgcataa actgatgaaa gatgacgttt 1080
atcgtggcta ccttatccca aaaaacacgc tggttttcgc gaacacttgg gctgtgctga 1140
acgatccgga ggtgtatccg gacccgtcgg tgttccgtcc ggagcgttat cttggcccgg 1200
acggcaaacc ggacaacacc gttcgtgatc cccgcaaggc tgcctttggt tacggtcgtc 1260
gtaattgccc gggcattcat ctggcacagt ctacggtgtg gattgccggt gcgaccctgc 1320
tgagcgcgtt caacattgaa agaccggttg atcaaaacgg caagccaatc gatatcccgg 1380
cggattttac caccggcttt ttccgccacc cggttccgtt ccagtgccgc tttgttccgc 1440
gtaccgagca ggtttctcag tccgtgtccg gtccgaagct t 1481
<210> 3
   <211> 1096
   <212> DNA
   <213> Psilocybe cubensis
<220>
   <223> 4-hydroxytryptamine kinase
<400> 3

ccatggcttt tgacctaaaa acagaagatg gacttatcac ctatctgacc aaacacctga 60
gcctggacgt cgatacctcc ggcgtgaagc gcctgagcgg cggtttcgtg aatgtgacgt 120
ggcgtatcaa gctgaacgct ccgtatcaag gacacacctc gatcatcctc aagcacgccc 180
agccacacat gagcacggac gaagatttta agatcggcgt ggaacgtagc gtgtatgaat 240
accaagcgat taagttaatg atggcgaatc gtgaagttct ggggggcgtc gatggtattg 300
tgagcgttcc ggagggtctg aactacgact tggaaaacaa cgcgctgatt atgcaggacg 360
tgggcaaaat gaaaaccttg ttggactacg tgacggctaa gccgcctctg gcaaccgaca 420
tcgcgcgctt ggttggcacc gagatcggcg gttttgttgc ccgcctgcat aacattggcc 480
gtgaacgccg tgatgatccg gagttcaaat tcttctccgg taacattgtt ggtcgtacca 540
cttcagatca gctgtaccaa accattatcc cgaatgccgc caagtacggc gttgatgacc 600
cgctgctccc gactgtcgtg aaggacttgg tcgacgacgt tatgcattcc gaggagacac 660
tggtcatggc agatttatgg tcaggcaaca tcctcttaca gcttgaggag ggcaacccga 720
gtaaactgca aaaaatttat atcctggact gggaactgtg caaatacggt ccggctagcc 780
tggacctggg ttatttcctg ggtgattgtt atctgattag ccgttttcag gatgagcagg 840
ttggcaccac catgcgtcag gcatacctgc aaagctatgc tcgcacctct aagcactcca 900
ttaattacgc aaaagttacg gcgggtatcg cggcgcatat tgtaatgtgg accgacttta 960
tgcagtgggg tagcgaagag gagcgtatca acttcgtgaa gaaaggtgtt gcggcgtttc 1020
acgatgcaag aggcaataac gataatggtg aaattacctc taccttgctt aaagaaagct 1080
ctacggcggc ggccgc 1096

SEQUENCE LISTING

<110> Synbionik GmbH

<120> Biosynthesis and Method of Production of Psilocin and Psilocybin in
Microorganisms

<130> SynPsy

<160> 3

<170> BiSSAP 1.3.6

<210> 1
<211> 1634
<212> DNA
<213> Gymnopilus dilepis

<220>
<223> Putative Dimethyltryptamine 4-hydroxylase

<400> 1
ccatgggccg tcgggcaaga agggctcgac tacccccggg tccaccgggt atcccgttgc 60

catttgtggg taatctgttt gatatgccgt ctaacagccc gtggctgacc tatctgcaat 120

ggggtgagac gtatcagacc gacattatct acctgaatgc gggtggcacc gagatggtga 180

ttcttaacac tttggaggcg attaccgatc tgttggagaa gagaggctcc atctatagcg 240

gtcgttttga aagcacgatg gtgaacgaat taatgggttg ggattttgac ttgggcttta 300

tcacgtacgg cgaacgctgg cgtgaggaac gtcgcatgtt cagcaaagag ttcaacgaga 360

agaacattaa gcagtttcgt cacgcccaga tccgtgctgc gaacttgctg gtcggccagc 420

tgaccaaaac cccggagcgt tggcatcaac tgatccgcca ccaaatagcg gcgatgtccc 480

tggatatcgg ttacggcatt gatctcttgg agggtgatcc gtggctggag gcgacccagt 540

tggcgaatga gggcctggcg atagcgagcg ttccgggtag cttctgggtt gattccctgc 600

cgattttaaa atacatgccc tcctggtttc cgggcgccga gttcaagcgc aaggcgaaag 660

tttggcgtga gagcaccgac cacatgatta acatgccgta cgaaaagatg aaaaagctga 720

tggttcaaga tttggttcgt ccgagttacg caagcgcgcg tctgcaagag atggacccga 780

atggcgatct gcagcatcaa gaacatgtta tccgtaacac cgcaatggaa gttaatgttg 840

gtggtgctga cactacagtg agcgcagtgg cggcgttcat cttagcgatg gtgaaatacc 900

cggacgttca gcgtaaggtg caggcagaac tggacgcggt tggctgtcgt gatgagctgc 960

cggagttcga cgaggacaac gatgcactgc cgtacctgac ggcgtgcgtt aaagaaattt 1020

ttcgttggaa tcaggtcgct ccgctggcca tcccgcaccg tctggacaaa gatgatcact 1080

atcgtggtta catcattccg aaaaacgcgc tggtgttcgc taatacttgg gcagtcctga 1140

atgacccatc ggtgtatccg gacccgtctg agttccgtcc ggagcgttat ctgggcccag 1200

acggcaagcc ggacccgcgt attcgcgacc cgcgtaaggc ggcgtttggt tatggcagac 1260

gcgcttgccc gggcatccac ctagctcaaa gcacggtgtg gattgttggt gctaccctgc 1320

tgtccgtgtt cgacatcgaa cgccctatgg atgcgaacgg taaaccgatt gacatcccag 1380

ctgcattcac caccggtttc tttcgctatt ctattcatga ttgtctcgtg gtggaaacga 1440

tgcacccggc gaacaccgtt tgcgtcgaca tcccgaaccc gtcggatgcc gatagcttcc 1500

tggtgccgaa gcgcctgtcg aacccgcatc cgattatcga cttgccgagc cgaaatccgg 1560

cgtgccagga agacggcgta gtggccttgt ctaacgcctg gcgcagcacc ttgccggttc 1620

aagacgtgaa gctt 1634

<210> 2
<211> 1481
<212> DNA
<213> Psilocybe cubensis

<220>
<223> Tryptamine 4-monooxygenase

<400> 2
ccatgggccg tcgggtacga aggtcaagac taccccctgg accgcctggc atcccgattc 60

catttatcgg taatatgttt gatatgcccg aggaaagccc gtggctgacc tttctgcaat 120

ggggtcgtga ttacaacacg gacatcctgt atgtggacgc gggcggtaca gaaatggtca 180

tcttgaacac cctggaaacc attaccgatt tgttggagaa acgcggtagc atttattccg 240

gcagattgga gagcacgatg gtgaacgagt tgatgggttg ggagttcgac ctgggcttca 300

ttacgtacgg cgaccgctgg cgtgaagagc gccgcatgtt cgccaaagag ttcagcgaaa 360

agggtatcaa acagtttcgt cacgcacaag taaaggcggc acatcaactg gtgcagcagt 420

tgaccaaaac gccggaccgc tgggcgcaac atattcgtca ccaaatcgct gccatgtctc 480

tcgatatcgg ctacggcatc gacttggcgg aagacgaccc gtggcttgag gcaacccatc 540

tggcgaatga aggtctggcg attgcgagcg ttccaggcaa gttctgggtc gactcgtttc 600

cgagcttaaa atacttaccg gcatggtttc cgggtgcggt gttcaaacgt aaggcgaaag 660

tttggcgtga agcagctgac cacatggtgg acatgccgta cgaaaccatg cgtaagctgg 720

ctccgcaggg tctgacccgt ccgagctatg caagcgcgcg tctccaggcg atggatctca 780

acggcgatct ggagcaccag gaacacgtga ttaagaacac tgcggcggag gttaatgttg 840

gtggcggtga taccaccgtt tccgccatgt cagcgttcat cctggctatg gtgaagtacc 900

cggaagtgca acgtaaggtt caagcggagc tggatgctct gacaaataac ggtcagatcc 960

cggactacga cgaagaggac gatagcctgc cgtatttgac tgcgtgtatt aaggagttgt 1020

ttcgctggaa tcagatcgca ccgctggcca tcccgcataa actgatgaaa gatgacgttt 1080

atcgtggcta ccttatccca aaaaacacgc tggttttcgc gaacacttgg gctgtgctga 1140

acgatccgga ggtgtatccg gacccgtcgg tgttccgtcc ggagcgttat cttggcccgg 1200

acggcaaacc ggacaacacc gttcgtgatc cccgcaaggc tgcctttggt tacggtcgtc 1260

gtaattgccc gggcattcat ctggcacagt ctacggtgtg gattgccggt gcgaccctgc 1320

tgagcgcgtt caacattgaa agaccggttg atcaaaacgg caagccaatc gatatcccgg 1380

cggattttac caccggcttt ttccgccacc cggttccgtt ccagtgccgc tttgttccgc 1440

gtaccgagca ggtttctcag tccgtgtccg gtccgaagct t 1481

<210> 3
<211> 1096
<212> DNA
<213> Psilocybe cubensis

<220>
<223> 4-hydroxytryptamine kinase

<400> 3
ccatggcttt tgacctaaaa acagaagatg gacttatcac ctatctgacc aaacacctga 60

gcctggacgt cgatacctcc ggcgtgaagc gcctgagcgg cggtttcgtg aatgtgacgt 120

ggcgtatcaa gctgaacgct ccgtatcaag gacacacctc gatcatcctc aagcacgccc 180

agccacacat gagcacggac gaagatttta agatcggcgt ggaacgtagc gtgtatgaat 240

accaagcgat taagttaatg atggcgaatc gtgaagttct ggggggcgtc gatggtattg 300

tgagcgttcc ggagggtctg aactacgact tggaaaacaa cgcgctgatt atgcaggacg 360

tgggcaaaat gaaaaccttg ttggactacg tgacggctaa gccgcctctg gcaaccgaca 420

tcgcgcgctt ggttggcacc gagatcggcg gttttgttgc ccgcctgcat aacattggcc 480

gtgaacgccg tgatgatccg gagttcaaat tcttctccgg taacattgtt ggtcgtacca 540

cttcagatca gctgtaccaa accattatcc cgaatgccgc caagtacggc gttgatgacc 600

cgctgctccc gactgtcgtg aaggacttgg tcgacgacgt tatgcattcc gaggagacac 660

tggtcatggc agatttatgg tcaggcaaca tcctcttaca gcttgaggag ggcaacccga 720

gtaaactgca aaaaatttat atcctggact gggaactgtg caaatacggt ccggctagcc 780

tggacctggg ttatttcctg ggtgattgtt atctgattag ccgttttcag gatgagcagg 840

ttggcaccac catgcgtcag gcatacctgc aaagctatgc tcgcacctct aagcactcca 900

ttaattacgc aaaagttacg gcgggtatcg cggcgcatat tgtaatgtgg accgacttta 960

tgcagtgggg tagcgaagag gagcgtatca acttcgtgaa gaaaggtgtt gcggcgtttc 1020

acgatgcaag aggcaataac gataatggtg aaattacctc taccttgctt aaagaaagct 1080

ctacggcggc ggccgc 1096

## Description

### BACKGROUND OF THE INVENTION

Psilocybin ([3-[2-(dimethylamino) ethyl]-1-methylindol-4-yl] dihydrogen phosphate) is a naturally occurring prodrug compound produced by fungi species, P. azurescens, P. semilanceate and P. cyanescens As a prodrug psilocybin is converted into psilocin (4-OH-DMT), one of the tryptamines, through biological omnipresent esterases. For all pharmaceutical purposes, psilocybin and psilocin can be assumed as equivalents.
Psilocybin and psilocin are the focus of multiple promising researches for experimental medical therapies. Recently, numerous studies have shown the efficiency of psilocin against psychological disorders like depression, anxiety and substance dependency. Depression is a major public health problem that affects approximately 4.4% of the global population (World Health Organization. Depression and other common mental disorders:global health estimates.Vol. 99. Geneva. (2017) p. 124-30. Retrieved from https://apps.who.int/). There potential use of these compounds in the treatment of those illnesses is very promising and would assemble the first new main structure and mode of action since decades in this field. Therefore FDA has claimed the Psilocybin treatment a "breakthrough therapy".Psilocin acts as a 5-HT agonist and has a particularly high affinity for the 5-HT2A receptor subtype, which is thought to be responsible for its psychotropic effects (David E. NicholsPharmacological Reviews April 1, 2016, 68 (2) 264-355; DOI: https://doi.org/10.1124/pr.115.011478). Addiction is a major concern associated with the use of psychoactive substances. However, one of the major benefits of these substances, psilocin and psilocybin, is that there is no strong evidence that suggest physical dependency (Johnson MW, Griffiths RR, Hendricks PS, Henningfield JE. The abuse potential of medical psilocybin according to the 8 factors of the Controlled Substances Act. Neuropharmacology. 2018;142:143-166. doi:10.1016/j.neuropharm.2018.05.012) . Psilocin and psilocybin have been the subject of deep research due to its potential positive effects against conditions such as anxiety, obsessive compulsive disorder and substance dependency (tabaco and alcohol). It has been registered to promote long-lasting positive changes in well-being, attitude, and personality upon a single administration (Mason, N. L., Mischler, E., Uthaug, M. V., & Kuypers, K. P. C. (2019). Subacute effects of psilocybin on empathy, creative thinking, and subjective well-being. Journal of Psychoactive Drugs. Advance online publication. https://doi.org/10.1080/02791072.2019.1580804).

Currently the mainstream source of psilocybinis harvesting for consumption of the popular denominated "magic mushrooms". However, it is extremely difficult to cultivate mushrooms under regulated conditions to produce a homogenous and consistent supply of medicine meeting Good Manufacturing Practices (GMP) criteria. In particular, the potency of "magic mushrooms" is not consistent and especially not high in concentration. Therefore, extraction from the natural source would be needed to get a reliable product. On the other hand, it is not trivial to scale up the production of mushrooms since the process depends on a 2D setup, limiting the use of space for production, and also depends on manual labor for cultivation and harvesting.

Therefore, a biological process eliminating those issues is needed to produce psilocin and psilocybin in larger scales avoiding the disadvantages of cultivation or chemical synthesis (use of transition metal chemistry, reactive species like reductive agents, water free conditions, dependency to fossil resources, etc.). Additionally, for these substances to be eligible candidates for the use in mainstream treatment, GMP and pharmaceutical standards must be met (e.g. quality control, sanitation, avoidance of cross contamination, free of human contaminants, etc...). Supply of the mushrooms in their natural form would not meet these requirements. Plus, this method would not be able to supply to a large population nor meet requirements to sustain transport nor exportation. Therefore, in order to meet the demands, cultivation would have to be done locally, requiring more resources and safety measures, cumbersome processes and extended timeframe.

US7229784B2 describes methods for improving the production of a secondary metabolite by a fungus by increasing the yield or productivity of the secondary metabolite produced by the fungus in the fungal cell. One of the preferred second metabolite is psilocybin. However, this method doesn't propose a specific scalable solution for the production of psilocybin. Additionally, compared to procaryotes, the manipulation of eukaryotic cells tends to be more complicated and generate more mutations when changes in their genome are performed. Moreover, this method would require further research and development in order to fully characterize and understand the genetic modification. Furthermore, this invention claims the need of utilizing antibacterial components such as penicillin. As it is common knowledge, penicillin is a strong and broad-spectrum antibiotic which traces in the final product could cause allergic reaction to possible patients. Additional studies must be carried out to confirm that no antibiotic substance is present in the final product and, if present, that it does not interfere with psilocybin to cause a harmful reaction to the consumer. This makes this supply methods more of a whole development project rather than a solution to the present problem.

US3192111A describes the preparation of psilocin and psilocybin using mushrooms as raw material in combination with other substances. This method does not propose a reliable solution to the problem described as it uses fungi as starting material.

The production of psilocin and psilocybin in modified microorganisms is a promising approach eliminating most of the problems related to growing fungi. The prior art proposes methodsto generate psilocin in microorganisms which resemble the natural pathway of psilocin biosynthesis that can be naturally found in mushrooms from genera Copelandia, Gymnopilus, Inocybe, Panaeolus, Pholiotina, Pluteus, and Psilocybe.

However, the present invention proposes an alternative route to the production of final product by changing the order of chemical transformations of the intermediates and adapting the enzyme activity in order to change substrate behavior. Importantly, the method of the invention enables to use natural occurring Dimethyltryptamin (DMT) as starting material., DMT is widely available by means of extraction from numerous natural sources (plant genera such as Phalaris, Delosperma, Acacia, Desmodium, Mimosa, Virola, Psychotria and from leaves, seeds, and inner bark of mimosa tenuiflora) and can therefore be used as an advantageous and economical precursor for the generation of the 4-hydroxylated derivate, psilocin (4-OH-DMT). Additionally, other higher substituted derivates of 4-OH-DMT, like 4-OH-MET or 4-OH-MiPT can be generated by simply using the hydroxylated tryptamines as starting materials. With slightly modified activity, hydroxylated tryptamines may also be of interest as potential therapeutic substances due to their interaction with the serotonergic system, which plays a role in the regulation of a variety of basic biological functions including sleep, appetite, circadian rhythm, and cognitive function. While generation of non-substituted tryptamines using either reductive alkylation of tryptamine or speeter anthony chemistry, or a combination of both to get asymmetric substituted tryptamines, is relatively easy, the same reactions using the 4-OH-indole system requires protection chemistry and the use of more expensive or even commercially unavailable compounds (.Chem. Rev. 2019, 119, 23, 11857-11911, October 2019; https://doi.org/10.1021/acs.chemrev.9b00383; Brandt, Simon & Freeman, Sally & Fleet, Ian & McGagh, Peter & Alder, John. (2004), The Analyst. 129. 1047-57. 10.1039.).

The method and sequences of the invention offer a simple alternative for 4-hydroxilation of tryptamines (e.g. DMT) .The inventors have also found that the use of anaerobic or facultative anaerobic organisms for the biosynthesis of psilocin reduces the oxidative degradation of psilocin, one of the main issues of a process involving psilocin.

### BRIEF SUMMARY OF THE INVENTION

The present invention relates generally to methods of production of psilocin and psilocybin, as well as expression of enzymes and recombinant microorganisms for the biosynthesis of pharmaceutical approved psilocin and psilocybin. Using DMT as starting material, heterologous enzymes are used to direct psilocin and/or psilocybin biosynthesis in modified microorganisms, e.g., bacteria, yeasts.

In one aspect, the present invention concerns an expression cassette comprising a heterologous promoter operably linked to a nucleic acid Putative Dimethyltryptamine 4-hydroxylase.

In another aspect, the present invention relates to an expression cassette comprising a heterologous promoter operably linked to a nucleic acid encoding Tryptamine 4-monooxygenase.

In another aspect, the present inventioni relates to an expression cassette comprising a heterologous promoter operably linked to a nucleic acid encoding 4-hydroxytryptamine kinase.

In a further aspect, the present invention concerns an expression cassette, wherein the putative Dimethyltryptamine 4-hydroxylase comprises a nucleotide sequence at least 60% identical to amino acid sequence of SEQ ID_NO 1.

In a further aspect, the present invention concerns the expression cassette, wherein the Tryptamine 4-monooxygenase comprises a nucleotide sequence at least 60% identical to amino acid sequence of SEQ ID_NO 2.

In a further aspect, the present invention concerns the expression cassette, wherein the 4-hydroxytryptamine kinase comprises a nucleotide sequence at least 60% identical to amino acid sequence of SEQ ID_NO 3.

In a further aspect, the present invention relates to the expression cassette, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is a constitutive promoter.

In a further aspect, the present invention describes the expression cassette, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is a inducible promoter.

In a further aspect, the present invention describes the expression cassette, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is a codon optimized promoter.

In a further aspect, the present invention describes the expression, wherein the Putative Dimethyltryptamine 4-hydroxylase is from Gymnopilus dilepis, Tryptamine 4-monooxygenase and 4-hydroxytryptamine kinase enzyme are from Psilocybe cubensis

In a further aspect, the present invention describes the plasmid, wherein the expression cassette further comprises an expression cassette comprising a nucleic acid encoding one or more, two or more, or all of the enzymes of the psilocybin and/or psilocin biosynthesis pathway.

In some embodiments, the present invention describes the plasmid, wherein the plasmid further comprises an expression cassette comprising a nucleic acid encoding one or more, two or more, or all of the enzymes of the psilocybin and/or psilocin biosynthesis pathway

In some embodiments, the present invention describes a host cell comprising:
a) An expression cassette comprising a heterologous promoter operably linked to a nucleic acid Putative Dimethyltryptamine 4-hydroxylase; and
b) an expression cassette further comprising a heterologous promoter operably linked to a nucleic acid encoding Tryptamine 4-monooxygenase

In some embodiments, the present invention describes a host cell comprising: a. An expression cassette comprising a heterologous promoter operably linked to a nucleic acid Putative Dimethyltryptamine 4-hydroxylase; and/or b) an expression cassette comprising a heterologous promoter operably linked to a nucleic acid encoding Tryptamine 4-monooxygenase; and/or c) an expression cassette comprising a heterologous promoter operably linked to a nucleic acid encoding 4-hydroxytryptamine kinase.

In a preferred embodiment, the host cell comprises the expression comprising a heterologous promoter operably linked to a nucleic acid Putative Dimethyltryptamine 4-hydroxylase; and b) an expression cassette comprising a heterologous promoter operably linked to a nucleic acid encoding Tryptamine 4-monooxygenase. In another preferred embodiment the host cell comprises a heterologous promoter operably linked to a nucleic acid Putative Dimethyltryptamine 4-hydroxylase; and b) an expression cassette comprising a heterologous promoter operably linked to a nucleic acid encoding Tryptamine 4-monooxygenase; and c) an expression cassette comprising a heterologous promoter operably linked to a nucleic acid encoding 4-hydroxytryptamine kinase.

In various embodiments the present invention describes the host cell, wherein the cell is selected from Escherichia, Saccharomyces, Clostridium, Bacillus, Lactococcus, Zymomonas, Corynebacterium, Pichia, Candida, Hansenula, Trichoderma, Acetobacterium, Ralstonia, Cupravidor, Salmonella, Klebsiella, Paenibacillus, Pseudomonas, Lactobacillus, Rhodococcus, Enterococcus, Alkaligenes, Brevibacterium, Methylobacterium, Methylococcus, Methylomonas, Methylocystis, Methylosinus, Clostridium, Moorella, Oxobacter, Acetobacterium, Eubacterium or Butyribacterium.

In various embodiments the present invention describes the host cell, wherein the cell is Zymomonas mobilis or Escherichia coli BL21 (D4-hydroxytryptamine kinase).

In various embodiments the present invention describes the host cell, wherein the expression cassette of a), b) and/or c) is integrated into the genome of the host cell.

In various embodiments the present invention describes the host cell, wherein the expression cassette of a),b) and/or c) are present in a single plasmid or inserted into a genome of the host cell at a single locus.

In various embodiments the present invention describes the host cell, wherein the expression cassette of a), b) and/or the expression cassette of c) are in different plasmids or inserted into a genome of the host cell at different loci.

In alternative embodiments the present invention describes a method of converting a precursor product such as DMT into a target metabolic product,such as psilocybin and/or psilocin the method comprising culturing a host cell in a suitable culture medium under conditions suitable to induce expression in one or more host cell expression cassettes of the invention, and then harvesting the cultured cells or spent medium, thereby converting the precursor product into the target metabolic product.

In alternative embodiments the present invention describes the method, wherein the starting product is Dimethyltryptamine

In alternative embodiments the present invention describes the method, wherein the metabolic product is psilocybin and/or psilocin.

In alternative embodiments the present invention describes the method, wherein the method comprises harvesting and lysing the cultured cells, thereby producing cell lysate.
In alternative embodiments the present invention describes the method, wherein the method comprises purifying the target metabolic product from the cell lysate, thereby producing a purified target metabolic product.

In alternative embodiments the present invention describes the method, wherein the method comprises purifying the target metabolic product from the spent culture medium, thereby producing a purified target metabolic product.

In alternative embodiments the present invention describes the method, wherein the purified target metabolic product is psilocin and the method comprises formulating psilocin in a pharmaceutical composition.

In alternative embodiments the present invention describes the method, wherein the purified target metabolic product is psilocybin and the method comprises formulating psilocybin in a pharmaceutical composition.

The invention is based, in part, on the discovery that the psilocin and psilocybin pathway can be efficiently expressed in microorganisms, such as bacteria or yeast, and utilized for production of psilocin and psilocybin. The invention also provides methods for expressing the psilocin and psilocybin pathway in microorganisms.

More specifically, the present invention relates to a method of production of psilocin and psilocybin in microorganisms, such as *Escherichia coli,* comprising the heterologous expression of the Putative Dimethyltryptamine 4-hydroxylasePutative Dimethyltryptamine 4-hydroxylase enzyme.

Provided herein are expression cassette comprising at least one of the genes encoding one of more enzymes of the psilocin and psilocybin pathway. The invention also relates to plasmids and microorganisms used for psilocin and psilocybin production. In some embodiments, provided is a recombinant expression cassette for producing psilocin or psilocybin in a cell, the recombinant expression cassette comprising a nucleic acid coding sequence of at least one enzyme of the psilocin and psilocybin pathway selected from the group comprising of

SEQ ID_NO 1 (Putative Dimethyltryptamine 4-hydroxylase):
CCATGGGCCGTCGGGCAAGAAGGGCTCGACTACCCCCGGGTCCACCGGGTAT
CCCGTTGCCATTTGTGGGTAATCTGTTTGATATGCCGTCTAACAGCCCGTGGC
TGACCTATCTGCAATGGGGTGAGACGTATCAGACCGACATTATCTACCTGAAT
GCGGGTGGCACCGAGATGGTGATTCTTAACACTTTGGAGGCGATTACCGATCT
GTTGGAGAAGAGAGGCTCCATCTATAGCGGTCGTTTGAAAGCACGATGGTG
AACGAATTAATGGGTTGGGATTTTGACTTGGGCTTTATCACGTACGGCGAACG
CTGGCGTGAGGAACGTCGCATGTTCAGCAAAGAGTTCAACGAGAAGAACATT
AAGCAGTTTCGTCACGCCCAGATCCGTGCTGCGAACTTGCTGGTCGGCCAGCT
GACCAAAACCCCGGAGCGTTGGCATCAACTGATCCGCCACCAAATAGCGGCG
ATGTCCCTGGATATCGGTTACGGCATTGATCTCTTGGAGGGTGATCCGTGGCT
GGAGGCGACCCAGTTGGCGAATGAGGGCCTGGCGATAGCGAGCGTTCCGGGT
AGCTTCTGGGTTGATTCCCTGCCGATTTTAAAATACATGCCCTCCTGGTTTCCG
GGCGCCGAGTTCAAGCGCAAGGCGAAAGTTTGGCGTGAGAGCACCGACCACA
TGATTAACATGCCGTACGAAAAGATGAAAAAGCTGATGGTTCAAGATTTGGT
TCGTCCGAGTTACGCAAGCGCGCGTCTGCAAGAGATGGACCCGAATGGCGAT
CTGCAGCATCAAGAACATGTTATCCGTAACACCGCAATGGAAGTTAATGTTG
GTGGTGCTGACACTACAGTGAGCGCAGTGGCGGCGTTCATCTTAGCGATGGT
GAAATACCCGGACGTTCAGCGTAAGGTGCAGGCAGAACTGGACGCGGTTGGC
TGTCGTGATGAGCTGCCGGAGTTCGACGAGGACAACGATGCACTGCCGTACC

TGACGGCGTGCGTTAAAGAAATTTTCGTTGGAATCAGGTCGCTCCGCTGGCC
ATCCCGCACCGTCTGGACAAAGATGATCACTATCGTGGTTACATCATTCCGAA
AAACGCGCTGGTGTTCGCTAATACTTGGGCAGTCCTGAATGACCCATCGGTGT
ATCCGGACCCGTCTGAGTTCCGTCCGGAGCGTTATCTGGGCCCAGACGGCAA
GCCGGACCCGCGTATTCGCGACCCGCGTAAGGCGGCGTTTGGTTATGGCAGA
CGCGCTTGCCCGGGCATCCACCTAGCTCAAAGCACGGTGTGGATTGTTGGTGC
TACCCTGCTGTCCGTGTTCGACATCGAACGCCCTATGGATGCGAACGGTAAAC
CGATTGACATCCCAGCTGCATTCACCACCGGTTTCTTTCGCTATTCTATTCATG
ATTGTCTCGTGGTGGAAACGATGCACCCGGCGAACACCGTTTGCGTCGACATC
CCGAACCCGTCGGATGCCGATAGCTTCCTGGTGCCGAAGCGCCTGTCGAACCC
GCATCCGATTATCGACTTGCCGAGCCGAAATCCGGCGTGCCAGGAAGACGGC
GTAGTGGCCTTGTCTAACGCCTGGCGCAGCACCTTGCCGGTTCAAGACGTGAA
GCTT

SEQ ID_NO 2 (Tryptamine 4-monooxygenase):
CCATGGGCCGTCGGGTACGAAGGTCAAGACTACCCCCTGGACCGCCTGGCAT
CCCGATTCCATTTATCGGTAATATGTTTGATATGCCCGAGGAAAGCCCGTGGC
TGACCTTTCTGCAATGGGGTCGTGATTACAACACGGACATCCTGTATGTGGAC
GCGGGCGGTACAGAAATGGTCATCTTGAACACCCTGGAAACCATTACCGATT
TGTTGGAGAAACGCGGTAGCATTTATTCCGGCAGATTGGAGAGCACGATGGT
GAACGAGTTGATGGGTTGGGAGTTCGACCTGGGCTTCATTACGTACGGCGAC
CGCTGGCGTGAAGAGCGCCGCATGTTCGCCAAAGAGTTCAGCGAAAAGGGTA
TCAAACAGTTTCGTCACGCACAAGTAAAGGCGGCACATCAACTGGTGCAGCA
GTTGACCAAAACGCCGGACCGCTGGGCGCAACATATTCGTCACCAAATCGCT
GCCATGTCTCTCGATATCGGCTACGGCATCGACTTGGCGGAAGACGACCCGTG
GCTTGAGGCAACCCATCTGGCGAATGAAGGTCTGGCGATTGCGAGCGTTCCA
GGCAAGTTCTGGGTCGACTCGTTTCCGAGCTTAAAATACTTACCGGCATGGTT
TCCGGGTGCGGTGTTCAAACGTAAGGCGAAAGTTTGGCGTGAAGCAGCTGAC
CACATGGTGGACATGCCGTACGAAACCATGCGTAAGCTGGCTCCGCAGGGTC
TGACCCGTCCGAGCTATGCAAGCGCGCGTCTCCAGGCGATGGATCTCAACGG
CGATCTGGAGCACCAGGAACACGTGATTAAGAACACTGCGGCGGAGGTTAAT
GTTGGTGGCGGTGATACCACCGTTTCCGCCATGTCAGCGTTCATCCTGGCTAT
GGTGAAGTACCCGGAAGTGCAACGTAAGGTTCAAGCGGAGCTGGATGCTCTG
ACAAATAACGGTCAGATCCCGGACTACGACGAAGAGGACGATAGCCTGCCGT
ATTTGACTGCGTGTATTAAGGAGTTGTTTCGCTGGAATCAGATCGCACCGCTG
GCCATCCCGCATAAACTGATGAAAGATGACGTTTATCGTGGCTACCTTATCCC
AAAAAACACGCTGGTTTTCGCGAACACTTGGGCTGTGCTGAACGATCCGGAG
GTGTATCCGGACCCGTCGGTGTTCCGTCCGGAGCGTTATCTTGGCCCGGACGG
CAAACCGGACAACACCGTTCGTGATCCCCGCAAGGCTGCCTTTGGTTACGGTC
GTCGTAATTGCCCGGGCATTCATCTGGCACAGTCTACGGTGTGGATTGCCGGT
GCGACCCTGCTGAGCGCGTTCAACATTGAAAGACCGGTTGATCAAAACGGCA
AGCCAATCGATATCCCGGCGGATTTTACCACCGGCTTTTTCCGCCACCCGGTT

CCGTTCCAGTGCCGCTTTGTTCCGCGTACCGAGCAGGTTTCTCAGTCCGTGTCC
GGTCCGAAGCTT

SEQ ID_NO 3 (4-hydroxytryptamine kinase):
CCATGGCTTTTGACCTAAAAACAGAAGATGGACTTATCACCTATCTGACCAAA
CACCTGAGCCTGGACGTCGATACCTCCGGCGTGAAGCGCCTGAGCGGCGGTT
TCGTGAATGTGACGTGGCGTATCAAGCTGAACGCTCCGTATCAAGGACACAC
CTCGATCATCCTCAAGCACGCCCAGCCACACATGAGCACGGACGAAGATTTT
AAGATCGGCGTGGAACGTAGCGTGTATGAATACCAAGCGATTAAGTTAATGA
TGGCGAATCGTGAAGTTCTGGGGGGCGTCGATGGTATTGTGAGCGTTCCGGA
GGGTCTGAACTACGACTTGGAAAACAACGCGCTGATTATGCAGGACGTGGGC
AAAATGAAAACCTTGTTGGACTACGTGACGGCTAAGCCGCCTCTGGCAACCG
ACATCGCGCGCTTGGTTGGCACCGAGATCGGCGGTTTTGTTGCCCGCCTGCAT
AACATTGGCCGTGAACGCCGTGATGATCCGGAGTTCAAATTCTTCTCCGGTAA
CATTGTTGGTCGTACCACTTCAGATCAGCTGTACCAAACCATTATCCCGAATG
CCGCCAAGTACGGCGTTGATGACCCGCTGCTCCCGACTGTCGTGAAGGACTTG
GTCGACGACGTTATGCATTCCGAGGAGACACTGGTCATGGCAGATTTATGGTC
AGGCAACATCCTCTTACAGCTTGAGGAGGGCAACCCGAGTAAACTGCAAAAA
ATTTATATCCTGGACTGGGAACTGTGCAAATACGGTCCGGCTAGCCTGGACCT
GGGTTATTTCCTGGGTGATTGTTATCTGATTAGCCGTTTTCAGGATGAGCAGG
TTGGCACCACCATGCGTCAGGCATACCTGCAAAGCTATGCTCGCACCTCTAAG
CACTCCATTAATTACGCAAAAGTTACGGCGGGTATCGCGGCGCATATTGTAAT
GTGGACCGACTTTATGCAGTGGGGTAGCGAAGAGGAGCGTATCAACTTCGTG
AAGAAAGGTGTTGCGGCGTTTCACGATGCAAGAGGCAATAACGATAATGGTG
AAATTACCTCTACCTTGCTTAAAGAAAGCTCTACGGCGGCGGCCGC

The invention describes the biosynthetic production of psilocin from the precursor molecule Dimethyltryptamine (DMT). This is enabled by the overexpression of putative Dimethyltryptamine 4-hydroxylaseand Tryptamine 4-monooxygenase used to convert Dimethyltryptamine (DMT) to psilocin. The heterologous genes used to the transformation are from *Gymnopilus dilepis,* a species of mushroom in the family Cortinariaceae (Putative Dimethyltryptamine 4-hydroxylase) and *Psilocybe cubensis,* species of psychedelic mushroom whose principal active compounds are psilocin and psilocybin (Tryptamine 4-monooxygenase).

The invention describes the biosynthetic production of psilocin from the precursor molecule psilocybin. This is enabled by the overexpression of 4-hydroxytryptamine kinase used to convert psilocybin to psilocin. The heterologous genes (4-hydroxytryptamine kinase) used to transform are from *Psilocybe cubensis,* species of psychedelic mushroom whose principal active compounds are psilocin and psilocybin.

Member(s) of the psilocin or psilocybin pathway refers to one or more enzymes or any polypeptides with enzymatic activities of the natural process (in fungus) of production of the compound of interest, in particular psilocin and psilocybin.

In some embodiments, the recombinant expression cassette includes nucleic acid coding sequence for at least 2, 3 or 4 members of the psilocin and psilocybin pathway, wherein each nucleic acid coding sequence is preceded by promoters such as T7, pBAD, endogenous promoters, lac, lacUV5, tac, Psyn, trp, araBAD, lpp^{a}, lpp-lac, phoA, recA, phoA, recA, proU, cst-1, testA,cadA, nar, P_{L}, cspA, T7-lac operator, T3-lac operator, T5-lac operator, T4 gene 32, nprM-lac operator, Vhb or Protein A.

In some embodiments, where the recombinant expression cassette comprises nucleic acid coding sequence for 2 or more members of the psilocin and psilocybin pathway, the 2 or more nucleic acid coding sequences are included on a single transcript upon expression (e.g., expression of the 2 or more nucleic acid coding sequences is driven by a single promotor and they are part of a single operon).

In some embodiments, the recombinant expression cassette comprises the nucleic acid coding sequence of Putative Dimethyltryptamine 4-hydroxylase and Tryptamine 4-monooxygenase, wherein the coding sequence is preceded by, and wherein each nucleic acid coding sequence is included on a single transcript upon expression (e.g., expression of the nucleic acid coding sequences is driven by a single promoter and they are part of a single operon).

In some embodiments, the nucleic acid coding sequences are arranged on the recombinant expression cassette in any order. In some embodiments, the nucleic acid coding sequences are arranged in the following order:
5'-Putative Dimethyltryptamine 4-hydroxylase-Tryptamine 4-monooxygenase-3'

In some embodiments, the RBS comprises at least 3, 4, 5, 6, 7, or 8 contiguous nucleotides complementary to the 16S rRNA (for expression in a prokaryotic cell) or the 18S rRNA (for expression in a eukaryotic cell). In some embodiments, the RBS comprises at least 3, 4, 5, 6, 7, or 8 contiguous nucleotides complementary to the I6S rRNA of bacteria (e.g., E. coli)). In some embodiments, the RBS is selected from any of the RBS sequences shown in SEQ ID NOs 1, 2 and 3.

In some embodiments, the nucleic acid coding sequence of Putative Dimethyltryptamine 4-hydroxylase encodes a polypeptide having at least 60, 65, 70, 75, 80, 85, 90, 95, 96, 97, 98, 99, or higher percent identity to SEQ ID NO 1, or a species homolog thereof, wherein the Putative Dimethyltryptamine 4-hydroxylase polypeptide retains Putative Dimethyltryptamine 4-hydroxylase activity. In some embodiments, the Putative Dimethyltryptamine 4-hydroxylase polypeptide has a sequence of SEQ ID NO 1. In some embodiments, the nucleic acid coding sequence of Putative Dimethyltryptamine 4-hydroxylase is codon-optimized for expression in a host cell. The Putative Dimethyltryptamine 4-hydroxylase gene transcription and Putative Dimethyltryptamine 4-hydroxylase translation and activity can be determined by transcriptome, proteome and metabolome analysis known in the art. In particular, the activity of the Putative Dimethyltryptamine 4-hydroxylase polypeptides or any variants can be tested.

In some embodiments, the nucleic acid coding sequence of Tryptamine 4-monooxygenase encodes a polypeptide having at least 60, 65, 70, 75, 80, 85, 90, 95, 96, 97, 98, 99, or higher percent identity to SEQ ID NO 2, or a species homolog thereof, wherein the Tryptamine 4-monooxygenase polypeptide retains Tryptamine 4-monooxygenase activity. In some embodiments, the Tryptamine 4-monooxygenase polypeptide has a sequence of SEQ ID NO 2. In some embodiments, the nucleic acid coding sequence of Tryptamine 4-monooxygenase is codon-optimized for expression in a host cell. The Tryptamine 4-monooxygenase gene transcription and Tryptamine 4-monooxygenase translation and activity can be determined by transcriptome, proteome and metabolome analysis known in the art. In particular, the activity of the Tryptamine 4-monooxygenase polypeptides or any variants can be tested.

In some embodiments, the nucleic acid coding sequence of 4-hydroxytryptamine kinase encodes a polypeptide having at least 60, 65, 70, 75, 80, 85, 90, 95, 96, 97, 98, 99, or higher percent identity to SEQ ID NO 3, or a species homolog thereof, wherein the 4-hydroxytryptamine kinase polypeptide retains 4-hydroxytryptamine kinase activity. In some embodiments, the 4-hydroxytryptamine kinase polypeptide has a sequence of SEQ ID NO 3. In some embodiments, the nucleic acid coding sequence of 4-hydroxytryptamine kinase is codon-optimized for expression in a host cell. The 4-hydroxytryptamine kinase gene transcription and 4-hydroxytryptamine kinase translation and activity can be determined by transcriptome, proteome and metabolome analysis known in the art. In particular, the activity of the 4-hydroxytryptamine kinase polypeptides or any variants can be tested.

In some embodiments, the recombinant expression cassette comprises a sequence having at least 80, 85, 90, 95, 96, 97, 98, 99, or higher percent identity to SEQ ID NO 1 and SEQ ID NO 2, wherein, upon expression in a cell, the cell produces more psilocin than a control cell lacking the recombinant expression cassette.

In some embodiments, the cell and control cell expressing Putative Dimethyltryptamine 4-hydroxylase and Tryptamine 4-monooxygenase further express heterologous 4-hydroxytryptamine kinase.

In some embodiments, the recombinant expression cassette comprises a single promoter driving expression of the at least one nucleic acid coding sequence, e.g, so that the nucleic acid coding sequences are included in a single operon. In some embodiments, the recombinant expression cassette comprises a single terminator. In some embodiments, the recombinant expression cassette comprises more than one promoter and/or more than one terminator. For example, the recombinant expression cassette can have a promoter driving expression of a first set of nucleic acid coding sequences, and a second promoter driving expression of a second set of nucleic acid coding sequences (and a third and fourth, etc.). In some embodiments, the recombinant expression cassette comprises a separate promoter for each nucleic acid coding sequence.

In some embodiments, the recombinant expression cassette comprises flanking regions, wherein a first flanking region is adjacent to, e.g., upstream of, the promoter and a second flanking region is adjacent to, e.g., downstream of, the terminator. In some embodiments, the first flanking region is preceded by a promoter. In some embodiments, the second flanking region is followed by a terminator. In some embodiments, the flanking regions are located on either side of, but not immediately adjacent to, the nucleic acid coding sequence(s) of the recombinant expression cassette.

In some embodiments, the flanking regions of the recombinant expression cassette comprise a nucleic acid sequences of a specific gene in the host cell. In some embodiments, the flanking regions of the recombinant expression cassette comprise sequences of a gene expressed in bacteria or yeast. For example, the flanking regions can be nucleic acid sequences of a gene, wherein the first and second flanking regions are not identical sequences. In some embodiments, the flanking regions contain sequences of a gene in expressed bacteria, such as, but not limited to restriction sites (e.g. Ncol, HindIII and Notl) enhancers, silencers, TATA box and CAAT box.

In some embodiments, the flanking regions are used for homologous recombination into the genome of a host cell, such as bacteria or yeast. In some embodiments, the flanking regions comprise a sequence having at least 50, 100, 200, 300, 400, 500, 600, or more base pairs of a gene in the host cell. For example, the flanking regions can be about 500 bp of sequence and located on either side of the transgene (including promoter, nucleic acid coding sequence(s), and terminator), wherein the sequence of the flanking regions are of a gene (i.e., a portion of a gene) present in the host cell. Non- limiting examples of genes in the host cell include restriction sites (e.g. Ncol, HindIII and Notl) enhancers, silencers, TATA box and CAAT box.

In some embodiments, the flanking regions comprise a nucleic acid sequences that can undergo double homologous recombination into the host cell's genome at a site of integration.

In some embodiments, the cell lacks the psilocin and psilocybin pathway, and thus lacks the ability to regulate psilocin and psilocybin production resulting from the psilocin and psilocybin pathway. Accordingly, further provided is a cell comprising the recombinant expression cassette as described above, wherein the cell lacks the psilocin and psilocybin pathway.

In some embodiments, the cell is selected from bacteria, yeast.

In various embodiments, the cell is a bacterium, and may be of a genus selected from the genus *Escherichia, Saccharomyces, Clostridium, Bacillus, Lactococcus, Zymomonas, Corynebacterium, Pichia, Candida, Hansenula, Trichoderma, Acetobacterium, Ralstonia, Cupravidor, Salmonella, Klebsiella, Paenibacillus, Pseudomonas, Lactobacillus, Rhodococcus, Enterococcus, Alkaligenes, Brevibacterium, Methylobacterium, Methylococcus, Methylomonas, Methylocystis* and *Methylosinus.*

In a preferred embodiment, the microorganism is selected from the group comprising of *Escherichia coli, Saccharomyces cerevisiae, Clostridium acetobutylicum, Clostridium beijerinckii, Clostridium saccharbutyricum, Clostridium saccharoperbutylacetonicum, Clostridium butyricum, Clostridium diolis, Clostridium kluyveri, Clostridium pasterianium, Clostridium novyi, Clostridium difficile, Clostridium thermocellum, Clostridium cellulolyticum, Clostridium cellulovorans, Clostridium phytofermentans, Lactococcus lactis, Bacillus subtilis, Bacillus licheniformis, Zymomonas mobilis, Klebsiella oxytoca, Klebsiella pneumonia, Corynebacterium glutamicum, Trichoderma reesei, Ralstonia eutropha, Cupriavidus necator, Pseudomonas putida, Lactobacillus plantarum* and *Methylob acterium extorquens.*

In another embodiment the genetically engineered microorganism is selected from a group comprising a carboxydotrophic bacteria from the genus *Clostridium, Moorella, Oxobacter, Acetobacterium, Eubacterium* or *Butyribacterium.*

In a preferred embodiment the carboxydotrophic microorganism is selected from a group comprising *Clostridium Ijungdahlii, Clostridium carboxydivorans, Clostridium ragsdalei, Clostridium autoethanogenum, Moorella thermoacetica, Moorella thermoautotrophica, Oxobacter pfennigii, Acetobacterium woodi, Eubacterium limosum* and *Butyribacterium methylotrophicum.*

In a preferred embodiment the microorganism is *Escherichia coli BLR (DE3)* as it is used for the heterologous expression of unstable proteins or enzymes (Goffin, Philippe, and Philippe Dehottay. "Complete genome sequence of Escherichia coli BLR (DE3), a recA-deficient derivative of E. coli BL21 (DE3)." Genome announcements 5.22 (2017)).

In a preferred embodiment the microorganism is *Escherichia coli Rosetta 2 (DE3)* as it is used for the heterologous expression of eukaryotic proteins or enzymes (Kopanic, Jennifer, et al. "An Escherichia coli strain for expression of the connexin45 carboxyl terminus attached to the 4th transmembrane domain." Frontiers in pharmacology 4 (2013): 106.).

In a preferred embodiment the microorganism is *Escherichia coli T7 Express* as it is an organism of choice for the general heterologous expression and production of recombinant proteins or enzymes (Lobstein, Julie, et al. "SHuffle, a novel Escherichia coli protein expression strain capable of correctly folding disulfide bonded proteins in its cytoplasm." Microbial cell factories 11.1 (2012): 753.).

In a preferred embodiment the microorganism is *Escherichia coli BL21 (DE3)* as it is an organism of choice for the heterologous expression and production of recombinant proteins or enzymes (Rosano, Germán L., and Eduardo A. Ceccarelli. "Recombinant protein expression in Escherichia coli: advances and challenges." Frontiers in microbiology 5 (2014): 172.).

In a preferred embodiment the microorganism is *Zymomonas mobilis* ZM4 as it comprises characteristics, for example containing the Entner-Doudoroff pathway, that allow the biosynthetic production of bio-products (e.g. wogonin) more suitable and efficient (He, Ming Xiong, et al. "Zymomonas mobilis: a novel platform for future biorefineries." Biotechnology for biofuels 7.1 (2014): 101.).

In a preferred embodiment the microorganism is *Saccharomyces Cerevisiae* as it is a eukaryotic microorganism and has advantages for the heterologous expression of eukaryotic (plant) enzymes. The robust cultivation and genetic modification of the microorganism is advantageous for metabolic engineering and expression of heterologous enzymes (Le Borgne, Sylvie. "Genetic engineering of industrial strains of Saccharomyces cerevisiae." Recombinant Gene Expression. Humana Press, Totowa, NJ, 2012. 451-465.).

In a preferred embodiment the microorganism is *Clostridium autoethanogenum* as it is an anaerobic microorganism. Therefore, it utilizes an alternative carbon source. The alternative carbon source for the fermentation reaction is a gaseous substrate containing at least one of CO, CO₂ and H₂. The substrate used can come from waste gas obtained as a by-product of an industrial process (for example steel manufacturing, gasification of biomass, coal, animal wastes, production of ferroalloys and municipal solid waste (Wu, Tongwei, et al. "Greatly improving electrochemical N2 reduction over TiO2 nanoparticles by iron doping." Angewandte Chemie International Edition 58.51 (2019): 18449-18453.)), or another source such as from automobile exhaust fumes (Xu Xu, Dan, et al. "Bacterial community and nitrate removal by simultaneous heterotrophic and autotrophic denitrification in a bioelectrochemically-assisted constructed wetland." Bioresource technology 245 (2017): 993-999.).

In some embodiments, the bacterium is *Escherichia coli* or *Zymomonas mobilis.* In various embodiments, the microbial cell is a yeast cell, which is a species of Saccharomyces, Pichia, or Yarrowia. For example, the microbial cell may be a species selected from Saccharomyces cerevisiae, Pichia pastoris, and Yarrowia lipolytica.

In some embodiments, the nucleic acid coding sequences are included on more than one recombinant expression cassette, e.g., 2, 3, or 4 recombinant expression cassettes. In some embodiments, the nucleic acid coding sequences are included on a single recombinant expression cassette in the cell. In various embodiments, two or more heterologous enzymes are expressed together in an operon, or are expressed individually. The enzymes may be expressed from extrachromosomal elements such as plasmids, or bacterial artificial chromosomes, or may be chromosomally integrated.

In some embodiments, the cell further comprises a nucleic acid coding sequence for Tryptamine 4-monooxygenase, wherein the SEQ ID _NO 2 coding sequence is on the same or a different recombinant expression cassette as the nucleic acid coding sequences for Putative Dimethyltryptamine 4-hydroxylase.

In some embodiments, the cell further comprises a nucleic acid coding sequence for 4-hydroxytryptamine kinase, wherein the SEQ ID_NO 3 coding sequence is on the same or a different recombinant expression cassette as the nucleic acid coding sequences for Putative Dimethyltryptamine 4-hydroxylase and Tryptamine 4-monooxygenase.

Further provided are methods for producing psilocin in a cell, e.g., a cell lacking the psilocin pathway. In some embodiments, the method comprises recombinantly expressing at least one other member of the psilocin pathway (i.e., phosphorylase or tryptophan decarboxylase) in the cell, and culturing the cell in the presence of Putative Dimethyltryptamine 4-hydroxylaseand Tryptamine 4-monooxygenase, thereby producing psilocin in the cell. In some embodiments, the method further comprises recombinantly expressing SEQ ID_NO 1 and 2.

In various embodiments, a recombinant host cell incorporates modifications that increase the intake of precursor, DMT, to enable high-titer production of psilocin or psilocybin.

In these or other embodiments, the host cell is modified for enhanced psilocin production. In some embodiments, a recombinant *Escherichia coli* cell overexpresses one or more enzymes of the psilocin pathway.

In these or other embodiments, the host cell is modified for enhanced psilocybin production. In some embodiments, a recombinant *Escherichia coli* cell overexpresses one or more enzymes of the psilocybin pathway.

In some embodiments, the culturing is carried out at 16-45°C, e.g., room temperature, 37°C, 30-42 °C, 30-40°C, or 32-38°C.

In some embodiments, Putative Dimethyltryptamine 4-hydroxylase, Tryptamine 4-monooxygenase and 4-hydroxytryptamine kinase are recombinantly expressed from at least one recombinant expression cassette as described above. For example, the recombinant expression cassette can comprise a nucleic acid coding sequence of at least one member of the psilocin and psilocybin pathway selected from the group consisting of phosphorylase or tryptophan decarboxylase, wherein the nucleic acid coding sequence of the at least one member of the psilocin and psilocybin pathway is preceded by a TIR comprising an RBS. In some embodiments, the recombinant expression cassette further comprises the nucleic acid coding sequence of Putative Dimethyltryptamine 4-hydroxylase and Tryptamine 4-monooxygenase preceded by a TIR comprising an RBS. In some embodiments, the recombinant expression cassette comprises the nucleic acid coding sequences of 4-hydroxytryptamine kinase. In some embodiments, the nucleic acid coding sequence of each member of the psilocin and psilocybin pathway is preceded by a TIR comprising an RBS. In some embodiments, each nucleic acid coding sequence is included on a single transcript upon expression. In some embodiments, the nucleic acid coding sequences are in the following order: 5'-Putative Dimethyltryptamine 4-hydroxylase-Tryptamine 4-monooxygenase-3'. In other embodiments, the nucleic acid coding sequences are in the following order: 5'-Tryptamine 4-monooxygenase-Putative Dimethyltryptamine 4-hydroxylase-3'. In other embodiments, the nucleic acid coding sequences are in the following order: 5'-Putative Dimethyltryptamine 4-hydroxylase-Tryptamine 4-monooxygenase-4-hydroxytryptamine kinase-3'. In other embodiments, the nucleic acid coding sequences are in the following order: 5'-Tryptamine 4-monooxygenase-Putative Dimethyltryptamine 4-hydroxylase-4-hydroxytryptamine kinase-3'.

In some embodiments, at least one recombinant expression cassette is targeted to the non-naturally occurring microorganisms' genome at a specific site of integration via double homologous recombination. In some instances, the site of integration includes any region of the host cell genome able to express a recombinant expression cassette. Sites of integration include but are not limited to, a neutral (Neu) site, PsbA2 gene (GenBank Accession no. X13547), GlgA gene, and GlgX gene.

In some embodiments, the method further comprises introducing the recombinant expression cassette to the cell prior to the expressing step. In some embodiments, the method further comprises introducing a recombinant expression cassette comprising Putative Dimethyltryptamine 4-hydroxylase and Tryptamine 4-monooxygenase, e.g., simultaneously or consecutively, with the recombinant expression cassette comprising the nucleic acid coding sequence of at least one member of the psilocin pathway.

In some embodiments, the method further comprises introducing the recombinant expression cassette to the cell prior to the expressing step. In some embodiments, the method further comprises introducing a recombinant expression cassette comprising Putative Dimethyltryptamine 4-hydroxylase, Tryptamine 4-monooxygenase and 4-hydroxytryptamine kinase, e.g., simultaneously or consecutively, with the recombinant expression cassette comprising the nucleic acid coding sequence of at least one member of the psilocybin pathway.

In some embodiments, the method further comprises harvesting the psilocin emitted from the cell.

In some embodiments, the method further comprises harvesting the psilocybin emitted from the cell.

One of skill will appreciate that, in practicing the method of producing psilocin or psilocybin in a cell, that the embodiments of the recombinant expression cassette and cell described above can be used. For example, members of the psilocin pathway can be included on more than one recombinant expression cassette, as described above, and that the TIRs and RBSs can be selected as described above. In addition, the recombinant expression cassette can have one or more promoters driving expression of the members of the psilocin pathway, and/or one or more terminators of transcription.

In some embodiments, the method results in an increase in the amount of psilocin produced by the cell compared to a control cell not recombinant' expressing the at least one member of the psilocin pathway. For example, the method results in at least a 2-, 5-, 6-, 8-, 10-, 20-, 30-, 40-, 50-, 60-, 70-, 80-, 90-, 100-, 200-, 500-, 600-, 1000-, 1200-, 1600-, 2000-fold or higher fold increase in the amount of psilocin produced by the cell compared to a control cell not recombinantly expressing the at least one member of the psilocin pathway.

In some embodiments, the method results in an increase in the amount of psilocybin produced by the cell compared to a control cell not recombinant' expressing the at least one member of the psilocybin pathway. For example, the method results in at least a 2-, 3-, 5-, 6-, 8-, 10, 20-, 30-, 40-, 50-, 60-, 70-, 80-, 90-, 100-, 200-,500-, 600-, 1000-, 1200-, 1600-, 2000-fold or higher fold increase in the amount of psilocybin produced by the cell compared to a control cell not recombinantly expressing the at least one member of the psilocybin pathway.

In some embodiments, the method results in a cessation of cell growth, i.e., the cells no longer replicate, or grow/ replicate at a greatly reduced rate. In some embodiments, the method results in cell growth 20, 10, 5, 1% or lower compared to a control cell not recombinantly expressing at least one member of the psilocin pathway. In some embodiments the precursor molecule, DMT, is quantitatively converted to psilocin, e.g., with an efficiency of 18, 25, 30, 50, 60, 70, 80% or higher. In some embodiments, the mass ratio of psilocin to dry cell weight (Isp/DCW) is at least 0.25, 0.5, 0.7, 0.8, 0.9, 1 .0 or higher. In some embodiments, the psilocin - to-biomass (w:w) ratio is at least 0.3%, 0.5%, 0.7%, 0.8%, 0.9%, 1.0%, 2.0%, 3.0%, 4.0%, 5.0%, 6.0%, 7.0%, 8.0%, 9.0%, 10.0%, 1 1.0%, 12.0%, 13.0%, 14.0%, 15.0% or more.

In some embodiments, the method results in a cessation of cell growth, i.e., the cells no longer replicate, or grow/ replicate at a greatly reduced rate. In some embodiments, the method results in cell growth 20, 10, 5, 1% or lower compared to a control cell not recombinantly expressing at least one member of the psilocybin pathway. In some embodiments the precursor molecule, psilocin, is quantitatively converted to psilocin, e.g., with an efficiency of 18, 25, 30, 50, 60, 70, 80% or higher. In some embodiments, the mass ratio of psilocin to dry cell weight (Isp/DCW) is at least 0.25, 0.5, 0.7, 0.8, 0.9, 1 .0 or higher. In some embodiments, the psilocybin -to-biomass (w:w) ratio is at least 0.3%, 0.5%, 0.7%, 0.8%, 0.9%, 1.0%, 2.0%, 3.0%, 4.0%, 5.0%, 6.0%, 7.0%, 8.0%, 9.0%, 10.0%, 1 1.0%, 12.0%, 13.0%, 14.0%, 15.0% or more.

In some aspects, the present invention provides methods for making a product comprising psilocin, psilocybin or psilocin and psilocybin. In various aspects, the product is a pharmaceutical composition, a dietary supplement or a baked good. The psilocin and psilocybin of the present invention can be mixed with one or more excipients to form a pharmaceutical product, which may be a pill, a capsule, a mouth spray, or an oral solution.

In an embodiment, shuttle vectors with the incorporated genes (Putative Dimethyltryptamine 4-hydroxylase and Tryptamine 4-monooxygenase or Putative Dimethyltryptamine 4-hydroxylase, Tryptamine 4-monooxygenase and 4-hydroxytryptamine kinase) are transformed into Zymomonas mobilis via electroporation and subsequently plated on agar plates containing one or more of the following antibiotics ampicillin, kanamycin, chloramphenicol, tetracycline and spectinomycin. Detection of the successful transformation of gene cassettes is accomplished via PCR and sequencing. The gene transcription and enzyme translation and activity are determined by transcriptome, proteome and metabolome analysis.

### DETAILED DESCRIPTION OF THE INVENTION

### Introduction

Psilocin and psilocybin, compounds of interest, are produced using an expression system as described herein that employs, as well as analogs of such compounds. In some embodiments, each step of a metabolic pathway that produces the psilocin or psilocybin, compound of interests, occurs in a modified recombinant cell described herein. In other embodiments, at least one step of the metabolic pathway occurs in a modified recombinant cell described herein, and at least one step of the metabolic pathway occurs extracellularly, e.g, in microorganism media or within a co-cultured modified recombinant cell.

The invention illustratively described herein suitably may be practiced in the absence of any element or elements, limitation or limitations that are not specifically disclosed herein. Thus, for example, in each instance herein any of the terms "comprising", "consisting essentially of" and "consisting of" may be replaced with either of the other two terms. Thus, for example, some embodiments may encompass a host cell "comprising" a number of components, other embodiments would encompass a host cell "consisting essentially of the same components, and still other embodiments would encompass a host cell "consisting of the same components. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims. The nucleic acid sequences provided by the invention is in the form of an expression cassette comprising a heterologous promoter operably linked to a nucleic acid encoding polypeptides being involved in psilocin and psilocybin synthesis.

The polynucleotides provided by the invention can either be isolated from their natural genomic environment, modified after their isolation, or produced artificially from pure sequence information. A natural source of polynucleotides of the invention are *Gymnopilus dilepis* and *Psilocybe cubensis.*

### Definitions

Unless otherwise defined, all terms of art, notations and other scientific terminology used herein are intended to have the meanings commonly understood by those of ordinary skill in the art to which the present application pertains. In some cases, terms with commonly understood meanings are defined herein for clarity and/or for ready reference, and the inclusion of such definitions herein should not necessarily be construed to represent a substantial difference over what is generally understood in the art.

As used herein and in the appended claims, the singular forms"a,""and," and"the" include plural referents unless the context clearly dictates otherwise.

The term "genome" or "genomic DNA" is referring to the heritable genetic information of a host organism. Said genomic DNA comprises the entire genetic material of a cell or an organism. In the case of eukaryotic organisms, the terms genome or genomic DNA refers to the total amount of DNA of a cell, including the DNA of the nucleus (chromosomal DNA), extrachromosomal DNA, and organellar DNA (e.g. of mitochondria). Preferably, the terms genome or genomic DNA, when used in context of eukaryotic organisms, is referring to the chromosomal DNA of the nucleus or genomic DNA or heterologous plasmid DNA.

The terms "nucleic acid," "polynucleotide," and "oligonucleotide" refer to a single or double-stranded polymer of deoxyribonucleotide or ribonucleotide bases read from the 5' to the 3' end. The monomer is typically referred to as a nucleotide. Nucleic acids can include modified nucleotides that permit correct read through by a polymerase and do not significantly alter expression of a polypeptide encoded by that nucleic acid.

As used herein, the terms"about" and"around" indicate a close range around a numerical value when used to modify that specific value. If"X" were the value, for example, "about X" or"around X" would indicate a value from 0.9X to 1.1X, e.g., a value from 0.95X to 1.05X, or a value from 0.98X to 1.02X, or a value from 0.99X to 1.01X. Any reference to "about X" or"around X" specifically indicates at least the values X, 0.9X, 0.91X, 0.92X, 0.93X, 0.94X, 0.95X, 0.96X, 0.97X, 0.98X, 0.99X, 1.01X, 1.02X, 1.03X, 1.04X, 1.05X, 1.06X, 1.07X, 1.08X, 1.09X, and 1.1X, and values within this range.

The phrase "nucleic acid sequence encoding" or a "nucleic acid coding sequence" refers to a nucleic acid which directs the expression of a specific protein or peptide. Such nucleic acid sequences include both the DNA strand sequence that is transcribed into RNA, and the RNA sequence that is translated into protein. The nucleic acid sequences include both the full length nucleic acid sequences as well as non-full length sequences derived from the full length sequences. In some embodiments, the nucleotide sequence is codon-optimized to reflect the typical codon usage of the host cell without altering the polypeptide encoded by the nucleotide sequence. In certain embodiments, the term "codon optimization" or "codon-optimized" refers to modifying the codon content of a nucleic acid sequence without modifying the sequence of the polypeptide encoded by the nucleic acid to enhance expression in a particular host cell. In certain embodiments, the term is meant to encompass modifying the codon content of a nucleic acid sequence as a means to control the level of expression of a polypeptide (e.g., either increase or decrease the level of expression). Accordingly, described are nucleic sequences encoding the enzymes involved in the engineered metabolic pathways. In some embodiments, a non-naturally occurring microorganism may express one or more polypeptide having an enzymatic activity necessary to perform the steps described below.

For example, a particular cell may comprises one, two, three, four, five or more than five nucleic acid sequences, each one encoding the polypeptide(s) necessary to produce psilocin or psilocybin compound, or compound intermediate described herein.

Alternatively, a single nucleic acid molecule can encode one, or more than one, polypeptide. For example, a single nucleic acid molecule can contain nucleic acid sequences that encode two, three, four or even five different polypeptides.

Nucleic acid sequences useful for the invention described herein may be obtained from a variety of sources such as, for example, amplification of cDNA sequences, DNA libraries, de novo synthesis, excision of genomic segment. The sequences obtained from such sources may then be modified using standard molecular biology and/or recombinant DNA technology to produce nucleic sequences having desired modifications. Exemplary methods for modification of nucleic acid sequences include, for example, site directed mutagenesis, PCR mutagenesis, deletion, insertion, substitution, swapping portions of the sequence using restriction enzymes, optionally in combination with ligation, homologous recombination, site specific recombination or various combination thereof. In other embodiments, the nucleic acid sequences may be a synthetic nucleic acid sequence. Synthetic polynucleotide sequences may be produced using a variety of methods described in U.S. Patent No. 7,323,320, as well as U.S. Pat. Appl. Pub. Nos. 2006/0160138 and 2007/0269870.

The term "promoter" refers to a polynucleotide which directs the transcription of a structural gene to produce mRNA. Typically, a promoter is located in the 5' region of a gene, proximal to the start codon of the coding region. If a promoter is an inducible promoter, then the rate of transcription increases in response to an inducing agent or by the induced release of a suppressor. In contrast, the rate of transcription is not regulated by an inducing agent, if the promoter is a constitutive promoter. A polynucleotide is "heterologous to" an organism or a second polynucleotide if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is not naturally associated with the promoter (e. g. a genetically engineered coding sequence or an allele from a different ecotype, variety or strain).

"Transgene", "transgenic" or "recombinant" refers to a polynucleotide manipulated by man or a copy or complement of a polynucleotide manipulated by man. For instance, a trans-genie expression cassette comprising a promoter operably linked to a second polynucleotide may include a promoter that is heterologous to the second polynucleotide as the result of manipulation by man (e.g., by methods described in Sambrook et al., Molecular Cloning- A Laboratory Manual, Cold Spring Harbor Laboratory, Cold Spring Harbor, New York, (1989) or Current Protocols in Molecular Biology Volumes 1 -3, John Wiley & Sons, Inc. (1994-1998)) of an isolated nucleic acid comprising the expression cassette. In another example, a recombinant expression cassette may comprise polynucleotides combined in such a way that the polynucleotides are extremely unlikely to be found in nature. For instance, restriction sites or plasmid vector sequences manipulated by man may flank or separate the promoter from the second polynucleotide. One of skill will recognize that polynucleotides can be manipulated in many ways and are not limited to the examples above.

Typically, the plasmid contains, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Zymomonas mobilis* Origin of Replication (OR) or Escherichia coli Origin or Replication (OR).

In case the term "recombinant" is used to specify an organism or cell, e.g. a microorganism, it is used to express that the organism or cell comprises at least one "transgene", "transgenic" or "recombinant" polynucleotide, which is usually specified later on. The terms "operable linkage" or "operably linked" are generally understood as meaning an arrangement in which a genetic control sequence, e.g. a promoter, enhancer or terminator, is capable of exerting its function with regard to a polynucleotide being operably linked to it, for example a polynucleotide encoding a polypeptide. Function, in this context, may mean for example control of the expression, i.e. transcription and/or translation, of the nucleic acid sequence. Control, in this context, encompasses for example initiating, increasing, governing, or suppressing the expression, i.e. transcription and, if appropriate, translation. Controlling, in turn, may be, for example, tissue and / or time specific. It may also be inducible, for example by certain chemicals, stress, pathogens and similar.

Preferably, operable linkage is understood as meaning for example the sequential arrangement of a promoter, of the nucleic acid sequence to be expressed and, if appropriate, further regulatory elements such as, for example, a terminator, in such a way that each of the regulatory elements can fulfill its function when the nucleic acid sequence is expressed. An operably linkage does not necessarily require a direct linkage in the chemical sense. Genetic control sequences such as, for example, enhancer sequences are also capable of exerting their function on the target sequence from positions located at a distance to the polynucleotide, which is operably linked. Preferred arrangements are those in which the nucleic acid sequence to be expressed is positioned after a sequence acting as promoter so that the two sequences are linked covalently to one another. The distance between the promoter and the amino acid sequence encoding polynucleotide in an expression cassette, is preferably less than 200 base pairs, especially preferably less than 100 base pairs, very especially preferably less than 50 base pairs. The skilled worker is familiar with a variety of ways in order to obtain such an expression cassette. However, an expression cassette may also be constructed in such a way that the nucleic acid sequence to be expressed is brought under the control of an endogenous genetic control element, for example an endogenous promoter, for example by means of homologous recombination or else by random insertion. Such constructs are likewise understood as being expression cassettes for the purposes of the invention.

The term "flanking regions" refers to regions or sequences located upstream and/or downstream of a nucleic acid coding sequence in a recombinant expression cassette which is involved in double homologous recombination (e.g., integration) of a portion of the cassette with a host cell's genome. The term "double homologous recombination" refers to the ability of nucleic acid sequences to exchange, wherein a nucleic acid stably integrates into the genome of a host cell's DNA sequence to make a new combination of DNA sequence.

The words "complementary" or "complementarity" refer to the ability of a nucleic acid in a polynucleotide to form a base pair with another nucleic acid in a second polynucleotide. For example, the sequence A-G-T is complementary to the sequence T-C-A. Complementarity can be partial, in which only some of the nucleic acids match according to base pairing, or complete, where ail the nucleic acids match according to base pairing.

The terms "protein", "peptide", and "polypeptide" are used interchangeably to denote an amino acid polymer or a set of two or more interacting or bound amino acid polymers. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid metics that function in a manner similar to the naturally occurring amino acids. Naturally occurring amino acids are those encoded by the genetic code, as well as those amino acids that are later modified, e.g., hydroxyproline, γ- carboxyglutaniate, and O-phosphoserme. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. The terms "non-naturally occurring amino acid" and "unnatural amino acid" refer to amino acid analogs, synthetic amino acids, and amino acid mimetics which are not found in nature.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

A "conservative" substitution as used herein refers to a substitution of an amino acid such that charge, hydrophobicity, and/or size of the side group chain is maintained. Illustrative sets of amino acids that may be substituted for one another include (i) positively- charged amino acids Lys, Arg and His; (ii) negatively charged amino acids Glu and Asp; (iii) aromatic amino acids Phe, Tyr and Trp; (iv) nitrogen ring amino acids His and Trp; (v) aliphatic amino acids Gly, Ala, Val, Leu and He; (vi) slightly polar amino acids Met and Cys; (vii) small-side chain amino acids Ser, Thr, Asp, Asn, Gly, Ala, Glu, Gin and Pro; (viii) small hydroxyl amino acids Ser and Thr; and sulfur-containing amino acids Cys and Met. Reference to the charge of an amino acid in this paragraph refers to the charge at pH 7.0.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Conservatively modified variants can include polymorphic variants, interspecies homologs (orthologs), intraspecies homologs (paralogs), and allelic variants.

The term "% identity" and its derivatives are used interchangeably herein with the term "% homology" and its derivatives to refer to the level of a nucleic acid or an amino acid sequence's identity between another nucleic acid sequence or any other polypeptides, or the polypeptide's amino acid sequence, where the sequences are aligned using a sequence alignment program. In the case of a nucleic acid the term also applies to the intronic and/or intergenic regions. The terms "identical" or % "identity," in the context of two or more nucleic acids or proteins, refer to two or more sequences or subsequences that are the same or have a specified percentage of nucleotides or amino acids that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters, or by manual alignment and visual inspection. See e.g. the NCBI web site at ncbi.nlm.nih.gov/BLAST/. Such sequences are then said to be "substantially identical." This definition also refers to, and can be applied to, the compliment of a test sequence. The definition also includes sequences that have deletions and/or additions, as well as those that have substitutions. Optimal alignment of such sequences can be carried out by any of the publically available algorithms or programs for determining sequence identity and alignment, e.g., BLAST. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimize alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1); 195-7).

The term "cassette" or "expression cassette" means those constructs in which the nucleic acid sequence encoding an amino acid sequence to be expressed is linked operably to at least one genetic control element which enables or regulates its expression (i.e. transcription and / or translation). An expression cassette typically includes a sequence to be expressed, and sequences necessary for expression of the sequence to be expressed. The sequence to be expressed can be a coding sequence or a non-coding sequence {e.g., an inhibitory sequence). Generally, an expression cassette is inserted into an expression vector (e.g., a plasmid) to be introduced into a host cell. The expression may be, for example, stable or transient, constitutive, or inducible. Expression cassettes may also comprise the coding regions for two or more polypeptides and lead to the transcription of polycistronic RNAs.

The term "effectively binds to ribosomes" or "effectively recruits ribosomes," in reference to an RBS, indicates that the RBS binds to ribosomes in the relevant cell or expression system in a manner sufficient to initiate translation. For example, an RBS in a bacterial (e.g., E. coli) cell is selected to bind to bacterial (Escherichia coli) ribosomes (e.g., the 16S rRNA), an RBS in a cyanobacterial cell (e.g., Synechocystis) is selected to bind to cyanobacterial ribosomes (e.g., the 16S rRNA) etc. One of skill will appreciate that the cell or expression system can be manipulated to include heterologous ribosomes that bind to a particular RBS.

The terms "transfection" and "transformation" refer to introduction of a nucleic acid into a cell by non-viral or viral-based methods. The nucleic acid molecules may be gene sequences encoding complete proteins or functional portions thereof. See, e.g., Sambrook ei ah, 1989, Molecular Cloning: A Laboratory Manual, 18.1- 18.88.

A polynucleotide or polypeptide sequence is "heterologous to" an organism or a second sequence if it originates from a different species, or, if from the same species, it is modified from its original form. For example, a promoter operability linked to a heterologous coding sequence refers to a coding sequence from a species different from that from which the promoter was derived, or, if from the same species, a coding sequence which is not naturally associated with the promoter (e.g. a genetically engineered coding sequence or an allele from a different ecotype or variety). Similarly, a heterologous expression cassette includes sequence!) that are from a different species than the cell into which the expression cassette is introduced, or if from the same species, is genetically modified. Yeast or other eukaryotic species may be introduced on high-level expression plasmid vectors or through genomic integration using methods well known to those skilled in the art. Such methods may involve CRISPR Cas-9 technology, yeast artificial chromosomes (YACs) or the use of retrotransposons. Alternatively, if natural to the host organism, such genes may be up regulated by genetic element integration methods known to those skilled in the art.

"Recombinant" refers to a genetically modified polynucleotide, polypeptide, cell, tissue, or organism. For example, a recombinant polynucleotide (or a copy or complement of a recombinant polynucleotide) is one that has been manipulated using well known methods. A recombinant expression cassette comprising a promoter operability linked to a second polynucleotide (e.g., a coding sequence) can include a promoter that is heterologous to the second polynucleotide as the result of human manipulation (e.g., by methods described in Sambrook ei αi, Molecular Cloning - A Laboratory Manual, Cold Spring Harbor Laboratory, Cold Spring Harbor, New York, (1989) or Current Protocols in Molecular Biology Volumes 1 -3, John Wiley & Sons, Inc. (1994- 1998)). A recombinant expression cassette (or expression vector) typically comprises polynucleotides in combinations that are not found in nature. For instance, human manipulated restriction sites or plasmid vector sequences can flank or separate the promoter from other sequences. A recombinant protein is one that is expressed from a recombinant polynucleotide, and recombinant cells, tissues, and organisms are those that comprise recombinant sequences (polynucleotide and/or polypeptide).

The terms "culture," "culturing," "grow," "growing," "maintain," "maintaining," "expand," "expanding," etc., when referring to cell culture itself or the process of culturing, can be used interchangeably to mean that a cell is maintained outside its normal environment under controlled conditions, e.g., under conditions suitable for survival. Cultured cells are allowed to survive, and culturing can result in cell growth, stasis, differentiation or division. The term does not imply that all cells in the culture survive, grow, or divide, as some may naturally die or senesce. Cells are typically cultured in media, which can be changed during the course of the culture.

The terms "media" and "culture solution" refer to the cell culture milieu. Media is typically an isotonic solution, and can be liquid, gelatinous, or semi-solid, e.g., to provide a matrix for cell adhesion or support. Media, as used herein, can include the components for nutritional, chemical, and structural support necessary for culturing a cell. Typically, media includes a carbon source for biosynthesis and metabolism. In the case of plant or other photosynthetic cell cultures, the carbon source is typically CO2.

A "control," e.g., a control cell, control sample, or control value, refers to a sample that serves as a reference, usually a known reference, for comparison to a test sample or condition. For example, a test sample can include cells exposed to a test condition or a test agent, while the control is not exposed to the test condition or agent (e.g., negative control). The control can also be a positive control, e.g., a known cell exposed to known conditions or agents, for the sake of comparison to the test condition. For example, a positive control can include a cell with a known level of production of the product of interest. A control can also represent an average value gathered from a plurality of samples, e.g, to obtain an average value. A control value can also be obtained from the same cell or population of cells, e.g., from an earlier-obtained sample, prior to the disorder or deficiency, or prior to treatment. One of skill will recognize that controls can be designed for assessment of any number of parameters. One of skill in the art will understand which controls are valuable in a given situation and be able to analyze data based on comparisons to control values. Controls are also valuable for determining the significance of data.

A tryptamine is a monoamine alkaloid, which contains an indole ring structure. It is structurally similar to the amino acid tryptophan, from which the name derives. Tryptamine is found in trace amounts in the brains of mammals and is hypothesized to play a role as a neuromodulator or neurotransmitter. Similar to other trace amines, tryptamine binds to human trace amine-associated receptor 1 (TAAR1) as an agonist. TAAR1 plays a significant role in regulating neurotransmission in dopamine, norepinephrine, and serotonin neurons in the CNS. Additionally, it also affects immune system and neuroimmune system function through different mechanisms. (Jones RS. Tryptamine: a neuromodulator or neurotransmitter in mammalian brain?. Prog Neurobiol. 1982;19(1-2):117-139. doi:10.1016/0301-0082(82)90023-5) (Khan MZ, Nawaz W. The emerging roles of human trace amines and human trace amine-associated receptors (hTAARs) in central nervous system. Biomed Pharmacother. 2016;83:439-449. doi:10.1016/j.biopha.2016.07.002) (Rogers TJ. The molecular basis for neuroimmune receptor signaling. J Neuroimmune Pharmacol. 2012;7(4):722-724. doi:10.1007/s11481-012-9398-4).

### RECOMBINANT METHODS FOR PRODUCTION OF PSILOCIN AND PSILOCYBIN

Methods of transformation of microorganisms such as bacteria or yeast cells are well known in the art.

Culture conditions such as expression time, temperature, and pH can be controlled so as to afford target psilocin and psilocybin in high yield. Host cells are generally cultured in the presence of starting materials, such as hexanoic acid, prenol, isoprenol, or the like, for periods of time ranging from a few hours to a day or longer (e.g, 24 hours, 30 hours, 36 hours, or 48 hours) at temperatures ranging from about 20 °C to about 40 °C depending on the particular host cells employed.

As explained above, in some embodiments, host cells, transformed or genomically integrated with plasmids or vectors containing at least one or more than one expression cassette.

The cells used to produce psilocin or psilocybin as described herein are genetically modified. That is, heterologous nucleic acid is introduced into the cells. The genetically modified cells do not occur in nature. Suitable cells are capable of expressing a nucleic acid construct (expression cassette) encoding biosynthetic enzymes, as described herein. In some embodiments, the cell naturally produces at least some biosynthetic precursors, e.g., DMT. In some embodiments, e.g., those involving psilocin or psilocybin production via the psilocin and psilocybin pathway, genes encoding desired enzymes can be heterologous to the cell, or native to the cell but operatively linked to heterologous promoters and/or control regions which result in the higher expression of the gene(s) in the cell.

Any microorganism can be used in the present method so long as it remains viable after being transformed with the heterologous genes.

Microorganisms used for producing psilocin or psilocybin, e.g., microorganisms lacking the psilocin and psilocybin pathway, e.g., bacteria, cyanobacteria or green microalgae, are engineered to express heterologous enzymes that generate psilocin and psilocybin.

Yeast or other eukaryotic species may be introduced on high-level expression plasmid vectors or through genomic integration using methods well known to those skilled in the art. Such methods may involve CRISPR Cas-9 technology, yeast artificial chromosomes (YACs) or the use of retrotransposons. Alternatively, if natural to the host organism, such genes may be up regulated by genetic element integration methods known to those skilled in the art.

The nucleic acid constructs described herein can be operably linked to a promoter and/or terminator so that the desired transcript(s) and protein(s) are expressed in a cell cultured under suitable conditions. Methods for designing and making nucleic acid constructs and expression vectors are well known to those skilled in the art.

Sequences of nucleic acids encoding the subject enzymes are prepared by any suitable method known to those of ordinary skill in the art, including, for example, direct chemical synthesis or cloning. For example, in direct chemical synthesis, oligonucleotides of up to about 40 bases are individually synthesized, then joined (e.g., by enzymatic or chemical ligation methods, or polymerase-mediated methods) to form essentially any desired continuous sequence. Further, commercial services are available that can supply synthetic genes of the desired sequence. In addition, the desired sequences may be isolated from natural sources using well known cloning methodology, e.g., employing PGR to amplify the desired sequences and join the amplified regions.

The nucleic acid coding sequences for desired biosynthetic enzymes can be incorporated into an expression cassette. Those of ordinary skill in the art are familiar with the necessary steps for incorporating a nucleic acid sequence into an expression cassette, and into an expression vector for introduction to a cell. A typical expression vector contains the desired nucleic acid sequence preceded by one or more regulatory regions (e.g., promoter), along with a ribosome binding site (RBS). Promoters can be either constitutive or inducible, e.g., under certain environmental conditions.

As will be appreciated by those of ordinary skill in the art, the invention is not limited with respect to the precise promoter or expression vector used. Although any suitable expression vector may be used to incorporate the desired sequences, readily available expression vectors include, without limitation: plasmids, such as pET, pGex, pJF119EH, pSC1O1, pBR322, pBBR1MCS-3, pUR, EX, pMRlOO, pCR4, pBAD24, pUC19; and bacteriophages, such as Ml 3 phage and λ phage. Certain expression vectors may only be suitable for particular host cells which can be readily determined by one of ordinary skill in the art. For example, the expression vector can be introduced into the host cell, which is then monitored for viability and expression of the sequences contained in the vector. In addition, reference may be made to the literature, which describe expression vectors and their suitability to any particular host cell.

Homologous recombination can occur between, the expression vector and the homologous region in one or more genomic copies present in the host cell. Typically, a selectable marker present on the expression vector is used to isolate transformant cells having undergone double homologous recombination by a selection method, such as antibiotic resistance or drug resistance.

Typically, the plasmid contains one or more specific promoters, one or more multiple cloning sites for the insertion of single genes or gene clusters, one or more terminators, one or more resistance cassettes (selected from ampicillin resistance (AmpR), kanamycin resistance (KanaR), chloramphenicol resistance (CamR), spectinomycin resistance (SpecR), tetracycline resistance (TetR)) and an *Zymomonas mobilis* Origin of Replication (OR) and *Escherichia coli* Origin of Replication.

Detection of the successful transformation of gene cassettes can be accomplished via PCR and sequencing. The gene transcription and enzyme translation and activity are determined by transcriptome, proteome and metabolome analysis.

Cell culture techniques are commonly known in the art and described, e.g., in Sambrook, et al. (1989) Molecular cloning : a laboratory manual, 2nd ed. Cold Spring Harbor Laboratory, Cold Spring Harbor, N.Y. Cells are typically cultured in isotonic media that includes a carbon source, and in some cases, selection factors to select for recombinant cells (e.g., those with antibiotic resistance).

The host cell is preferably cultured at a temperature between 22° C and 37° C. While commercial biosynthesis in host cells such as E. coli can be limited by the temperature at which overexpressed and/or foreign enzymes (e.g., enzymes derived from plants) are stable, recombinant enzymes (including the terpenoid synthase) may be engineered to allow for cultures to be maintained at higher temperatures, resulting in higher yields and higher overall productivity. In some embodiments, the host cell (bacterial or yeast host cell) is cultured at about 22° C or greater, about 23° C or greater, about 24° C or greater, about 25° C or greater, about 26° C or greater, about 27° C or greater, about 28° C or greater, about 29° C or greater, about 30° C or greater, about 31° C or greater, about 32° C or greater, about 33° C or greater, about 34° C or greater, about 35° C or greater, about 36° C or greater, or about 37° C.

Psilocin and psilocybin can be extracted from media and/or whole cells and recovered. The psilocin or psilocybin are recovered and optionally purified by fractionation (e.g. fractional distillation, chromatography, etc...). The product can be recovered by any suitable process, including partitioning the desired product into an organic phase. Various methods of psilocin and psilocybin preparation are known in the art, such as extraction from the fungus species. The production of the desired product can be determined and/or quantified, for example, by gas chromatography (e.g., GC) or high-pressure liquid chromatography (HPLC-)-.in combination with a suitable detection method, preferably mass spectrometry (MS) or other systems (FID, RI, DAD)

The desired product can be produced in batch or continuous bioreactor systems.

The amounts of psilocin and psilocybin can be measured in a recombinant host cell to identify rate limiting steps in the biosynthetic pathway. Once a rate-limiting step has been identified, expression or activity of the (limiting) one or more enzyme can be modified by various methods known in the art, such as codon optimization, use of a stronger or weaker promotor, expressing multiple copies of the corresponding gene, and constructing variants with increase stability and/or activity.

Identification and quantification of psilocin and psilocybin can be performed by several methods combining chromatographic separation; for example LC, HPLC, UHPLC or GC, and mass sensitive or photo optical detection. Chromatographic methods can include liquid (MeOH, ACN, hexan, water, acetic acid and others) or gaseous mobile phases (H₂, He, N₂, Ar) and liquid or solid stationary phases including silica gel, polydimethylsiloxane or reversed phase materials. Detection of psilocin and psilocybin and related compounds can be achieved by using MS or MS/MS including sector mass spectrometry, time-of-flight mass spectrometry, the use of quadrupole mass analyzer, three-dimensional quadrupole ion trap, cylindrical ion trap, linear quadrupole ion trap, orbitrap or fourier transform ion cyclotron resonance and the use of an andequat detector, including electron multiplier systems, faraday cups, ion-to-photon detectors, microchannel plate detectors or inductive detectors. Other detection methods like UV-absorption, fluorescence, charged aerosol detector, evaporative light scattering detector, flame ionization detector, flame photometric detector, nitrogen phosphorus detector, atomic-emission detector, refractive index detector, radio flow detector, conductivity monitor, thermal conductivity detector, electron capture detector and photoionization detectors or combination of those principles can be applied. Psilocin and psilocybin can also be detected by using chemical reactions, the use of appropriate stains like iodine vapor, iodoplatinate, marquis reagent, nihydrin, HNO₃-atmosphere, NNCD-reagent, PDAB-TS, TACOT, TCBI, vanillin reagents, Van Urk reagent or xanthydrol and the use of an authentic reference substance (Barker et al. 2012 DOI 10.1002/dta.422) (Mulga et al. 2007 ISBN 0-9770876-5-4).

Quantification can be achieved by the use of an internal or external standard containing psilocin and psilocybin or isotope labeled derivatives. By the addition of a known amount of these substances prior to sample preparation, it is possible to calculate the actual amount of psilocin and psilocybin and related compounds in each sample (Barker et al. 2013 DO110.1002/bmc.2981).

One or more psilocin and psilocybin may be produced by a recombinant host cell are partially or completely exported to the culture medium. In other embodiments, psilocin and psilocybin are produced simultaneously produced by a recombinant host cell are retained within the recombinant cell. Psilocin and psilocybin can be recovered from the culture medium or from the recombinant host cell.

The following examples are offered for illustrative purposes only and are not intended t o limit the scope of the present invention in any way. Indeed, various modifications of the invention in addition to those shown and described herein will become apparent to those skilled in the art from the foregoing description and fall within the scope of the appended claims.

## Claims

1. An expression cassette comprising a heterologous promoter operably linked to a nucleic acid Putative Dimethyltryptamine 4-hydroxylase.

2. An expression cassette of claim 1 further comprising a heterologous promoter operably linked to a nucleic acid encoding Tryptamine 4-monooxygenase.

3. An expression cassette of claim 1 and 2 further comprising a heterologous promoter operably linked to a nucleic acid encoding 4-hydroxytryptamine kinase.

4. The expression cassette of claim 1, wherein the Putative Dimethyltryptamine 4-hydroxylase comprises a nucleotide sequence at least 60% identical to amino acid sequence of SEQ ID_NO 1.

5. The expression cassette of claim 2, wherein the Tryptamine 4-monooxygenase comprises a nucleotide sequence at least 60% identical to amino acid sequence of SEQ ID_NO 2.

6. The expression cassette of claim 3, wherein the 4-hydroxytryptamine kinase comprises a nucleotide sequence at least 60% identical to amino acid sequence of SEQ ID_NO 3.

7. The expression cassette of any of claims 1-6, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is a constitutive promoter.

8. The expression cassette of any of claims 1-6, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is an inducible promoter.

9. The expression cassette of any of claims 1-6, wherein the promoter operably linked to the nucleic acid encoding the Putative Dimethyltryptamine 4-hydroxylase enzyme and/or to the nucleic acid encoding the Tryptamine 4-monooxygenase and/or 4-hydroxytryptamine kinase is a codon optimized promoter.

10. The expression cassette of any of the claims 1-9, wherein the sequence of Putative Dimethyltryptamine 4-hydroxylase is from Gymnopilus dilepis, and/or the sequence of Tryptamine 4-monooxygenase and/or 4-hydroxylase enzyme are from Psilocybe cubensis

11. The plasmid of claim 10, wherein the expression cassette further comprises an expression cassette comprising a nucleic acid encoding one or more, two or more, or all of the enzymes of the psilocybin and/or psilocin biosynthesis pathway

12. A plasmid comprising an expression cassette according to any one of claims 1-6.

13. The plasmid of claim 10, wherein the plasmid further comprises an expression cassette comprising a nucleic acid encoding one or more, two or more, or all of the enzymes of the psilocybin and/or psilocin biosynthesis pathway.

14. A host cell comprising any one of the expression cassettes of claims 1-10 and/or any one of the plasmids of claims 11-13.

15. A host cell comprising:
a. An expression cassette of claim 1; and
b. An expression cassette of claim 2

16. A host cell comprising:
c. further comprising an expression cassette of claim 3

17. The host cell of any one of claims 14-16, wherein the cell is selected from Escherichia, Saccharomyces, Clostridium, Bacillus, Lactococcus, Zymomonas, Corynebacterium, Pichia, Candida, Hansenula, Trichoderma, Acetobacterium, Ralstonia, Cupravidor, Salmonella, Klebsiella, Paenibacillus, Pseudomonas, Lactobacillus, Rhodococcus, Enterococcus, Alkaligenes, Brevibacterium, Methylobacterium, Methylococcus, Methylomonas, Methylocystis, Methylosinus, Clostridium, Moorella, Oxobacter, Acetobacterium, Eubacterium or Butyribacterium.

18. The host cell of claim 17, wherein the cell is Zymomonas mobilis or Escherichia coli BL21 (DE3).

19. The host cell of any one of claims 14-18, wherein the expression cassettes are integrated into the genome of the host cell.

20. The host cell of any one of claims 14-19, wherein the expression cassette are present in a single plasmid or inserted into a genome of the host cell at a single locus.

21. The host cell of any one of claims 14-20, wherein the expression cassettes are in different plasmids or inserted into a genome of the host cell at different loci.

22. A method of converting a precursor product into a target metabolic product, the method comprising culturing a host cell according to any one of claims 14-21 in a suitable culture medium under conditions suitable to induce expression in one or more host cell expression cassettes, and then harvesting the cultured cells or spent medium, thereby converting the precursor product into the target metabolic product.

23. The method of claim 22, wherein the starting product is Dimethyltryptamine

24. The method of claim 22, wherein the metabolic product is psilocybin and/or psilocin.

25. The method of any one of claims 22-24, wherein the method comprises harvesting and lysing the cultured cells, thereby producing cell lysate.

26. The method of claim 25, wherein the method comprises purifying the target metabolic product from the cell lysate, thereby producing a purified target metabolic product.

27. The method of any one of claims 25-26, wherein the method comprises purifying the target metabolic product from the spent culture medium, thereby producing a purified target metabolic product.

28. The method of claim 27, wherein the purified target metabolic product is psilocin

29. The method of claim 27, wherein the purified target metabolic product is psilocybin
